(19) Europäisches Patentamt
European Patent Office
Office européen des brevets

(11) Numéro de publication: 0 112 785 B1

# (12) FASCICULE DE BREVET EUROPEEN

(45) Date de publication du fascicule du brevet: **14.09.88**

(51) Int. Cl.[4]: **C 07 D 307/86**

(21) Numéro de dépôt: **83420173.3**

(22) Date de dépôt: **14.11.83**

(54) **Procédé de préparation du dihydro-2,3 diméthyl-2,2 hydroxy-7 benzofuranne.**

(30) Priorité: **18.11.82 FR 8219548**

(43) Date de publication de la demande: **04.07.84 Bulletin 84/27**

(45) Mention de la délivrance du brevet: **14.09.88 Bulletin 88/37**

(84) Etats contractants désignés: **AT BE CH DE FR GB IT LI LU NL SE**

(56) Documents cités:
**EP - A - 0 025 843**
**EP - A - 0 030 511**
**EP - A - 0 090 679**

(73) Titulaire: **RHONE-POULENC AGROCHIMIE, 14-20, rue Pierre Baizet, F-69009 Lyon (FR)**

(72) Inventeur: **Veracini, Serge, 36, Avenue de Ménival, F-69005 Lyon (FR)**

(74) Mandataire: **Brachotte, Charles et al, RHONE-POULENC AGROCHIMIE 14-20, Rue Pierre Baizet B.P. 9163, F-69263 Lyon Cedex 09 (FR)**

EP 0 112 785 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description pour les états contractants: AT, BE, CH, GB, LI, LU, NL, SE**

L'invention concerne un procédé de préparation du dihydro-2,3 diméthyl-2,2 hydroxy-7 benzofuranne à partir de l'orthométhallyloxyphénol.

Le dihydro-2,3 diméthyl-2,2 hydroxy-7 benzofuranne, désigné dans ce qui suit par DDHB, a pour formule:

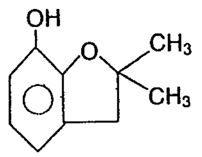

DDHB

Ce composé, en soi connu, est utilisable pour la préparation du méthylcarbamate de dihydro-2,3 diméthyl-2,2 benzofurannyl-7, insecticide polyvalent connu sous le nom de carbofuran.

L'orthométhallyloxyphénol, ou méthallyloxy-2 phénol, est le composé de formule:

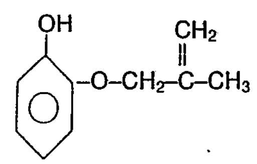

ORTHOMETHALLYLOXYPHENOL

Il est connu de préparer le DDHB à partir de l'orthométhallyloxyphénol par réarrangement de ce composé (transposition en ortho du radical méthallyle), formant intermédiairement un composé réarrangé (notamment orthométhallylpyrocatéchine) puis cyclisation de ce composé réarrangé, pour donner le DDHB.

L'orthométhallylpyrocatéchine, ou dihydroxy-1,2 méthallyl-3 benzène, est le composé de formule:

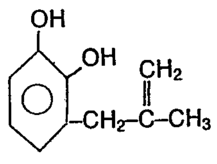

La mise en œuvre de cette transformation de l'orthométhallyloxyphénol en DDHB, par réarrangement/cyclisation a fait l'objet de plusieurs dépôts de brevets, conduisant à des résultats sensiblement différents selon les conditions utilisées:

– FR-A-1 403 952 décrit cette transformation effectuée par chauffage de l'orthométhallyloxyphénol, dans la masse, à 190 °C, la température atteignant 270 °C pendant la transformation. D'après l'essai décrit dans ce brevet le rendement n'est que de 48,2%;

– la demande de brevet européen no 79 420 062.6 (Numéro de publication: 0 012 096) décrit cette transformation effectuée par chauffage de l'orthométhallyloxyphénol à une température de 200 °C environ, en présence d'eau et éventuellement d'un solvant organique inerte. Les rendements obtenus sont de l'ordre de 69 à 72%;

– la demande de brevet européen no 80 420 130.9 (Numéro de publication 0 030 511) décrit cette transformation effectuée par chauffage de l'orthométhallyloxyphénol, à des températures en général de l'ordre de 120 à 150 °C, en présence, comme catalyseur, d'un dérivé de l'aluminium, de préférence l'isopropylate d'aluminium et éventuellement en présence d'un solvant organique. Les rendements obtenus varient de 60 à 77% environ. L'exemple 9 de cette demande de brevet européen montre que lorsqu'on opère à température plus basse (100 °C), on obtient un rendement en DDHB sensiblement plus faible (60,7%), malgré l'utilisation de quantités plus importantes de catalyseur (0,3 mole de catalyseur par mole d'orthométhallyloxyphénol);

– la demande de brevet européen no 80 104 521.2 (Numéro de publication 0 025 843) décrit cette transformation effectuée par chauffage de l'orthométhallyloxyphénol, à des températures de 150 à 190 °C environ, dans un solvant constitué par un polyhydroxyalkyléther contenant au moins un groupe OH, en présence, comme catalyseur, d'acide p-toluène-sulfonique ou de chlorure ferrique. Selon les exemples décrits, les rendements varient de 65 à 70%.

Aucun des procédés décrits dans ces références n'a toutefois donné pleinement satisfaction.

Un but de l'invention est de fournir un procédé amélioré de préparation du DDHB grâce à l'utilisation de catalyseurs différents de ceux décrits ou suggérés dans les brevets ou demandes de brevets cités plus haut.

Plus précisément, un but de la présente invention est de fournir un procédé de préparation du DDHB à partir de l'orthométhallyloxyphénol permettant d'obtenir de bons rendements en DDHB tout en opérant à une température sensiblement moins élevée que les procédés décrits dans les brevets ou demandes de brevets cités plus haut.

Il a maintenant été trouvé que ce but pouvait être atteint grâce à un nouveau procédé faisant l'objet de la présente invention.

Ce procédé concerne la préparation du DDHB à partir de l'orthométhallyloxyphénol, par chauffage, comportant un réarrangement de l'orthométhallyloxyphénol puis une cyclisation du composé réarrangé. Il est caractérisé en ce que au moins l'étape de cyclisation est effectuée par chauffage en présence d'une quantité efficace d'un catalyseur constitué par un dérivé de l'étain.

Selon un premier mode de réalisation de l'invention, l'orthométhallyloxyphénol est d'abord chauffé à une température élevée en l'absence de dérivé de l'étain et il se produit alors, selon les conditions utilisées, une conversion, partielle ou totale, du composé de départ en composé réarrangé (orthométhallylpyrocatéchine notamment). Le mélange réactionnel ainsi obtenu est ensuite chauffé en présence du dérivé de l'étain de manière à effectuer la cyclisation du dérivé réarrangé.

Selon ce premier mode de réalisation de l'invention, l'étape de réarrangement et l'étape de cyclisation peuvent être effectuées en deux opérations tout à fait distinctes: on peut ainsi chauffer l'orthométhallyloxyphénol de façon à provoquer son réarrangement, par exemple en opérant selon les conditions décrites dans la demande de brevet européen No. 80 420 130.9 (page 9 essai comparatif dans EP-A-30 511) et obtenir ainsi un mélange comprenant principalement de l'orthométhallylpyrocatéchine, puis dans une deuxième opération distincte de la précédente, chauffer ce mélange réactionnel en présence du dérivé de l'étain comme défini ci-dessus. Selon une variante de ce premier mode de réalisation de l'invention, on peut également effectuer successivement dans un même dispositif le réarrangement de la cyclisation en effectuant d'abord le chauffage en l'absence du dérivé de l'étain, puis en le poursuivant en présence du dérivé de l'étain que l'on ajoute alors au mélange réactionnel.

Selon un mode de réalisation préférentiel de l'invention, le réarrangement de l'orthométhallyloxyphénol s'effectue en chauffant ce composé en présence du dérivé de l'étain et la cyclisation du composé réarrangé est également effectuée par chauffage en présence du dérivé de l'ètain. Selon ce mode de réalisation préférentiel de l'invention, il est particulièrement avantageux de chauffer l'orthométhallyloxyphénol en présence du dérivé de l'étain et de poursuivre ce chauffage en présence du même dérivé de l'étain jusqu'à ce qu'il y ait formation du DDHB. Dans ces conditions, on observe que les deux transformations successives s'enchaînent immédiatement. On obtient ainsi un procédé permettant de produire le DDHB, en une seule étape, à partir de l'orthométhallyloxyphénol. Ce procédé est caractérisé en ce que l'orthométhallyloxyphénol est chauffé en présence d'un dérivé de l'étain. Il constitue le mode de réalisation préféré de l'invention.

Par dérivé de l'étain, au sens du présent texte, on entend les dérivés organiques ou minéraux de l'étain divalent et de l'étain tétravalent ainsi que tout réactif à base d'étain susceptible de former in situ, dans les conditions de la réaction un dérivé de l'étain divalent ou de l'étain tétravalent.

Ces dérivés de l'étain divalent et de l'étain tétravalent répondent à la formule générale:

$$Sn\ (R)_m$$

dans laquelle R représente un atome par exemple d'halogène, d'oxygène ou de soufre, ou un radical minéral ou organique, et m un nombre entier égal à 2 ou à 4 étant entendu que les substituants R peuvent être soit identiques soit différents.

Avantageusement R est choisi parmi les substituants mentionnés ci-après:

- les atomes d'halogène (de préférence le chlore),
- les radicaux alkyle (par exemple éthyle, méthyle, butyle) ou cycloalkyle éventuellement substitués (par exemple par un ou plusieurs halogènes),
- les radicaux aryle ou alkaryle (par exemple le radical phényle) éventuellement substitués,
- les radicaux $-OR_1$ dans lesquels $R_1$ représente un reste organique tel que par exemple un radical alkyle ou aryle ou acyle, ou le radical benzofuryle, ces radicaux pouvant être éventuellement substitués (pas exemple le radical dihydro-2,3 diméthyl-2,2 benzofuryle-7 ou le radical orthométhallyloxyphényle, ou les radicaux hydroxy(méthallyl)phényle, ces divers radicaux pouvant éventuellement être formés in situ).

Ainsi lorsque R représente le radical $-OR_1$, ce substiuant R peut, entre autres, signifier le radical phénoxy ou le radical acétyloxy ($CH_3COO$).

Parmi les composés répondant à la formule ci-dessus, on peut utiliser selon l'objet de l'invention avantageusement des composés tels que $SnCl_2$, le méthylate d'étain, $SnCl_4$, le diacétate d'étain, le diacétate de dibutylétain, le dichlorodibutylétain, cette liste n'étant pas limitative.

De préférence, on utilise comme dérivé de l'étain les dérivés de l'étain divalent, c'est-à-dire les composés répondant à la formule générale indiquée plus haut, dans laquelle m est égal à 2, R ayant la même signification que précédemment.

Parmi ces composés préférés, le chlorure stanneux $SnCl_2$ est tout particuliérement préféré, car il permet d'obtenir des rendements élevés dès la température de 100 °C, comme le montrent les exemples décrits plus bas.

La quantité de dérivé de l'étain à utiliser doit être suffisante pour permettre à la transformation de s'effectuer de façon satisfaisante dans les conditions utilisées. Pour cela on utilise généralement au moins 0,00005 mole de dérivé de l'étain par mole de composé à transformer (orthométhallyloxyphénol ou dérivé de réarrangement de ce composé). La limite supérieure de la quantité de catalyseur n'est pas critique. En pratique, on utilise avantageusement de 0,0001 à 0,3 mole de dérivé de l'étain par mole de composé à transformer et de préférence de 0,005 à 0,1 mole de dérivé de l'étain par mole de composé à transformer.

La température à laquelle est effectué le chauffage en présence du dérivé de l'étain peut être comprise entre 60 °C et 200 °C, elle est de préférence comprise entre 80 °C et 130 °C.

Lorsque le dérivé de l'étain utilisé comme catalyseur est le chlorure stanneux, des résultats tout à fait avantageux sont obtenus en opérant dès la température de 100 °C.

La transformation selon l'invention peut être effectuée par chauffage de l'orthométhallyloxyphénol et/ou de son produit de réarrangement dans la masse en l'absence de solvant.

De préférence toutefois, cette transformation est effectuée dans un solvant organique avantageusement choisi parmi les hydrocarbures aromatiques tels que le toluène, les o-, m- et p-xylènes; les hydrocarbures aliphatiques tels que l'octane ou le décane; les hydrocarbures aromatiques chlorés; les hydrocarbures cycloaliphatiques tels que le cyclohéxane; les éthers aliphatiques ou aromatiques tels que l'anisole; les cétones telles que la méthylisobutylcétone; les alcools tels que les alcanols inférieurs comme par exemple l'isopropanol, les phénols et plus particulièrement le phénol proprement dit, les polyhydroxyalkyléthers (tels que mentionnés dans la demande de

brevet européen no 80 104 521.2 = EP-A-25 843) cette liste n'étant pas limitative.

La transformation selon l'invention s'effectue avantageusement sous pression atmosphérique ordinaire. Elle peut toutefois être effectuée sous une pression différente de la pression atmosphérique ordinaire. Ainsi, lorsqu'on désire utiliser un solvant dont la température d'ébullition, sous pression atmosphérique normale, est inférieure à la température à laquelle on désire opérer, la transformation selon l'invention peut être effectuée en opérant en autoclave, sous une pression supérieure à la pression atmosphérique.

La durée nécessaire pour effectuer la transformation selon l'invention dépend de la température utilisée, ainsi que d'autres facteurs tels que le choix du catalyseur, mais en général cette durée est d'autant plus courte que la température est plus élevée. Cette durée est généralement comprise entre 15 minutes et vingt heures.

En fin de réaction, le DDHB obtenu est séparé par tout moyen connu en soi tel que par exemple par distillation. Toutefois, pour certaines utilisations, il peut ne pas être nécessaire d'isoler le DDHB et il suffit alors de le laisser dans le milieu réactionnel qui est lui-même mis en œuvre de la manière souhaitée. Le dérivé de l'étain peut être éliminé en lavant la masse réactionnelle par une solution aqueuse d'un acide fort.

Les exemples suivants, décrits à titre non limitatif, illustrent l'invention et montrent comment elle peut être mise en œuvre.

Dans ce qui suit, on désignera systématiquement par TT(ME) le taux de transformation de l'orthométhallyloxyphénol, évalué à partir de la quantité de ce composé présente dans le milieu réactionnel respectivement au début et à la fin de l'opération, et par RT(DDHB) le rendement en dihydro-2,3 diméthyl-2,2 hydroxy-7 benzofuranne (ou DDHB), calculé à partir de l'orthométhallyloxyphénol transformé.

Exemple 1

L'appareillage utilisé est constitué par un ballon de 100 ml, muni d'un réfrigérant ascendant et d'un barreau magnétique pour l'agitation.

On charge successivement dans ce ballon:

- 1,0294 g d'orthométhallyloxyphénol ($6,28.10^{-3}$ mole)
- 0,052 g de chlorure stanneux ($2,74.10^{-4}$ mole)
- 50 ml de méthylisobutylcétone.

Ce mélange est chauffé pendant 16 heures 30 à 100 °C, sous agitation, puis refroidi à température ambiante (25 °C). On ajoute alors 20 ml d'HCl 2N puis 20 ml d'acétate d'éthyle, et on agite l'ensemble dans une ampoule à décanter.

Après séparation de la phase organique et de la phase aqueuse, on lave la phase organique par 30 ml d'eau distillée que l'on réunit ensuite à la phase aqueuse. Cette phase aqueuse est à son tour lavée par 20 ml d'acétate d'éthyle qu'on joint à la première phase organique. On ajuste alors le volume de la phase organique à 100 ml et la solution obtenue est analysée par chromatographie en phase liquide.

On y dose:

- 0,0224 g d'orthométhallyloxyphénol non transformé ($1,36.10^{-4}$ mole)
- 0,793 g de DDHB ($4,83.10^{-3}$ mole)
- 0,0017 g d'orthométhallylpyrocatéchine ($1.10^{-5}$ mole).

Les résultats sont les suivants:

- TT(ME) = 98%
- RT(DDHB) = 78,7%.

Exemple 2

On opère selon la même méthode qu'à l'exemple 1 en utilisant le même dérivé d'étain ($SnCl_2$) et le même solvant que dans cet exemple 1, à partir de:

- 1,9421 g d'orthométhallyloxyphénol ($1,184.10^{-2}$ mole)
- 0,1121 g de chlorure stanneux ($5,91.10^{-4}$ mole)
- 35 ml de méthylisobutylcétone

et en effectuant le chauffage à 105 °C, comme à l'exemple no 1, mais pendant une durée de 9 heures 30.

Les résultats sont les suivants:

- TT(ME) = 100%
- RT(DDHB) = 79%.

Exemple 3

On opère selon la même méthode qu'à l'exemple 1, en utilisant le même dérivé d'étain et le même solvant que dans cet exemple 1, à partir de:

- 2,040 g d'orthométhallyloxyphénol ($12,44.10^{-3}$ mole)
- 0,101 g de chlorure stanneux ($5,3.10^{-4}$ mole)
- 18 ml de méthylisobutylcétone.

Le chauffage est effectué à 105 °C, pendant 6 heures, sous agitation, en atmosphère inerte.

Les résultats sont les suivants:

- TT(ME) = 87,7%
- RT(DDHB) = 79,3%.

Exemple 4

On opère selon la même méthode qu'à l'exemple 1 en utilisant le même dérivé d'étain qu'à l'exemple 1 mais en remplaçant la méthylisobutylcétone par l'isopropanol et en opérant dans un autoclave, sous atmosphère inerte.

L'essai est effectué en partant de:

- 1,619 g d'orthométhallyloxyphénol ($9,87.10^{-3}$ mole)
- 0,0891 g de chlorure stanneux ($0,470.10^{-3}$ mole)
- 12 ml d'isopropanol.

Le mélange est chauffé à 100 °C pendant 16 heures.

Les résultats sont les suivants:

- TT(ME) = 54,3%
- RT(DDHB) = 79,6%.

Exemple 5

On opère selon la même méthode qu'à l'exemple 1, en utilisant le même dérivé d'étain que dans cet exemple 1, mais en chauffant en masse l'orthométhallyloxyphénol, sans utilisation de solvant.

L'essai est effectué en partant de:

- 1,4727 g d'orthométhallyloxyphénol ($8,98.10^{-3}$ mole)
- 0,0150 g de chlorure stanneux ($7,9.10^{-5}$ mole).

Le chauffage est effectué à 100 °C pendant 7 heures 30.

Les résultats sont les suivants:

- TT(ME) = 100%
- RT(DDHB) = 53%.

Exemple 6

On opère selon la même méthode qu'à l'exemple 1, mais en utilisant comme catalyseur l'acétate d'étain, de formule:

$$(CH_3COO)_2\ Sn$$

et en opérant dans le m-yxlène comme solvant.

L'essai est effectué en partant de:

- 1,6456 g d'orthométhallyloxyphénol ($10,03.10^{-3}$ mole)
- 0,0487 g d'acétate d'étain ($0,206.10^{-3}$ mole)
- 11 ml de m-xylène.

Le chauffage est effectué à 120 °C, pendant 16 heures, sous atmosphère inerte.

Les résultats sont les suivants:

- TT(ME) = 86,6%
- RT(DDHB) = 67,3%

Exemple 7

On opère selon la même méthode qu'à l'exemple 1, mais en utilisant comme catalyseur le méthylate d'étain de formule:

$$(CH_3O)_2\ Sn$$

et en chauffant l'orthométhallyloxyphénol en masse, sans utilisation de solvant.

L'essai est effectué à partir de:

- 2,2740 g d'orthométhallyloxyphénol ($13,86.10^{-3}$ mole)
- 0,0273 g de méthylate d'étain ($0,15.10^{-3}$ mole).

Le chauffage est effectué à 97 °C, pendant 14 heures, sous atmosphère inerte, avec agitation.

Les résultats sont les suivants:

- TT(ME) = 63,5%
- RT(DDHB) = 61,7%.

Le méthylate d'étain a été préparé selon la méthode décrite par J.S. MORRISON et H.M. HAENDLER dans J. Inorg. Nucl. Chem. 29, 395 (1967).

Exemple 8

On opère selon la même méthode qu'à l'exemple 1, mais en utilisant comme catalyseur le diacétate de dibutylétain de formule:

$$(CH_3COO)_2\ Sn\ (C_4H_9)_2$$

et en chauffant en masse sans utilisation de solvant.

L'essai est effectué en partant de:

- 1,720 g d'orthométhallyloxyphénol ($10,49.10^{-3}$ mole)
- 0,0368 g de diacétate de dibutylétain ($0,105.10^{-3}$ mole).

Le chauffage est effectué à 95 °C pendant 15 heures 30, sous atmosphère inerte, avec agitation.

Les résultats sont les suivants:

- TT(ME): 56%
- RT(DDHB): 50%.

Exemple 9

On opère selon la même méthode qu'à l'exemple 1, mais en utilisant comme catalyseur le dichlorodibutylétain, de formule:

$$(Cl)_2\ Sn\ (C_4H_9)_2$$

et en chauffant en masse, sans utilisation de solvant.

L'essai est effectué en partant de:

- 2,020 g d'orthométhallyloxyphénol ($12,19.10^{-3}$ mole)
- 0,036 g de dichlorodibutylétain ($0,118.10^{-3}$ mole).

Le chauffage est effectué à 120 °C pendant 6 heures.

Les résultats sont les suivants:

- TT(ME): 67,5%
- RT(DDHB): 55,5%.

Exemple 10

On opère selon la même méthode qu'à l'exemple 1, mais en utilisant comme catalyseur le chlorure stannique de formule:

$$SnCl_4$$

et en opérant comme dans l'exemple 1 dans la méthylisobutylcétone.

L'essai est effectué à partir de:

- 2,020 g d'orthométhallyloxyphénol ($12,32.10^{-3}$ mole)
- 0,147 g de chlorure stannique ($0,56.10^{-3}$ mole)
- 18 ml de méthylisobutylcétone.

Le chauffage est effectué à 105 °C pendant 6 heures sous agitation et en atmosphère inerte.

Les résultats sont les suivants:

- TT(ME): 99%
- RT(DDHB): 48%.

Exemple 11

On utilise un tube muni d'une agitation et placé sous atmosphère inerte.

Dans ce tube on place 0,1336 g d'un produit contenant 80% en poids d'orthométhallylpyrocatéchine ($0,652.10^{-3}$ mole) et 0,0043 g de diacétate de dibutylétain ($0,012.10^{-3}$ mole).

Le chauffage est effectué pendant 3 heures 30 à 130 °C.

On observe alors que la totalité de l'orthométhallylpyrocatéchine a disparu du mélange réactionnel et on obtient ainsi 0,109 g de DDHB soit un rendement en DDHB calculé par rapport à l'orthométhallylpyrocatéchine de départ égal à 100%.

L'orthométhallylpyrocatéchine a été obtenue à partir d'orthométhallyloxyphénol par chauffage à 200 °C pendant 1 heure 30, dans le cyclohexane.

Essai A

Dans le même appareillage qu'à l'exemple 1, l'orthométhallyloxyphénol a été chauffé en masse, sans utilisation de solvant et en l'absence de dérivé de l'étain, à une température de 112 °C et de chauffage a été poursuivi à cette même température pendant 24 heures.

Les résultats sont les suivants:

- TT(ME): 43%
- RT(DDHB): 0%.

Une analyse du mélange réactionnel montre qu'il comprend principalement de l'orthométhallylpyrocatéchine (non cyclisée) et, dans une proportion moindre, de la paraméthallylpyrocatéchine (ou dihydroxy-1,2 méthallyl-4 benzène), respectivement 62,3% et 20,2% par rapport à l'orthométhallyloxyphénol transformé.

Essai B

On répète l'essai A, en chauffant en masse l'orthométhallyloxyphénol en l'absence de dérivé de l'étain mais en effectuant ce chauffage à 130 °C pendant 15 heures.

Les résultats sont les suivants:

- TT(ME): 82%
- RT(DDHB): 1,4%.

Une analyse du mélange réactionnel montre qu'il comprend principalement de l'orthométhallylpyrocatéchine (non cyclisée) et dans une proportion moindre de la paraméthallylpyrocatéchine, respectivement 61% et 22% par rapport à l'orthométhallyloxyphénol transformé.

Essai C

On répète l'essai A en chauffant l'orthométhallyloxyphénol en l'absence de dérivé de l'étain si ce n'est que l'orthométhallyloxyphénol est chauffé à 140 °C pendant 11 heures dans un solvant organique (para-xylène).

Les résultats sont les suivants:

- TT(ME): 98,2%
- RT(DDHB): 1,8%.

Une analyse du mélange réactionnel montre qu'il comprend principalement de l'orthométhallylpyrocatéchine (non cyclisée) et dans une proportion moindre de la paraméthallylpyrocatéchine.

Ces essais A, B et C montrent qu'en l'absence de dérivé de l'étain il n'y a pratiquement pas formation de DDHB pour des températures allant de 112 °C à 140 °C, puisque dans ces conditions le RT(DDHB) est compris entre 0% et 1,8%.

L'exemple C montre de plus que le réarrangement de l'orthométhallyloxyphénol peut s'effectuer de façon satisfaisante (TT-ME = 98,2%) en l'absence de catalyseur, à condition de chauffer à une température plus élevée (140 °C).

Les essais décrits dans les exemples 1 à 11 de notre demande de brevet montrent que dans des conditions de température tout à fait similaires, et dans certains cas plus basses, la présence d'un dérivé de l'étain dans le mélange réactionnel permet à la cyclisation de s'effectuer de façon satisfaisante, puisque dans ces conditions le RT(DDHB) varie de 48% (exemple 10) à 79,6% (exemple 4).

Une comparaison des résultats obtenus avec les dérivés de l'étain divalent, décrits dans les exemples no 1 à 7 de notre demande de brevet, à ceux obtenus avec les dérivés de l'étain tétravalent, et décrits dans les exemples no 8 à 10 de notre demande de brevet montre que les dérivés de l'étain divalent conduisent à des RT(DDHB) en général sensiblement plus élevés que les RT(DDHB) obtenus avec les dérivés de l'étain tétravalent.

Enfin, une comparaison du résultat obtenu avec $SnCl_2$, dans l'exemple 3: RT(DDHB) = 79,3%, à celui obtenu avec $SnCl_4$ dans l'exemple 10: RT(DDHB) = 48%, en utilisant exactement les mêmes conditions que dans l'exemple 3, met en évidence la nette supériorité de $SnCl_2$ vis-à-vis de $SnCl_4$ pour la transformation de l'orthométhallyloxyphénol en DDHB.

Par ailleurs, si l'on se réfère aux exemples no 1 et 2 de notre demande de brevet, on observera que l'utilisation de $SnCl_2$ comme catalyseur permet d'obtenir, dès la température de 100 °C, à la fois un taux de transformation de l'orthométhallyloxyphénol élevé: TT(ME) de 98% à 100% et un RT(DDHB) élevé (78,7% à 79%), c'est-à-dire des résultats sensiblement équivalents aux meilleurs résultats obtenus à des températures nettement plus élevées dans les exemples décrits dans les publications EP-A-0 012 096 et 0 030 511 citées plus haut.

**Revendications pour les états contractants: AT, BE, CH, GB, LI, LU, NL, SE**

1. Procédé de préparation du dihydro-2,3 diméthyl-2,2 hydroxy-7 benzofuranne par chauffage, à partir de l'orthométhallyloxyphénol, par réarrangement de l'orthométhallyloxyphénol, puis cyclisation du composé, réarrangé ainsi obtenu, caractérisé en ce que au moins l'étape de cyclisation est effectuée par chauffage en présence d'une quantité efficace d'un catalyseur constitué par un dérivé de l'étain.

2. Procédé selon la revendication 1, caractérisé en ce que le réarrangement de l'orthométhallyloxyphénol est effectué par chauffage en l'absence de dérivé de l'étain puis en ce que la cyclisation du composé réarrangé est effectuée par chauffage de ce composé en présence d'un catalyseur constitué par un dérivé de l'étain.

3. Procédé selon l'une des revendications 1 et 2, caractérisé en ce que le composé réarrangé puis soumis à cyclisation est l'orthomethallylpyrocatéchine.

4. Procédé selon la revendication 1, caractérisé en ce que le réarrangement de l'orthométhallyloxyphénol est effectué par chauffage en présence d'un dérivé de l'étain, puis en ce que la cyclisation du composé réarrangé est effectuée par chauffage en présence d'un dérivé de l'étain.

5. Procédé selon la revendication 4, caractérisé en ce que l'orthométhallyloxyphénol est chauffé en présence d'un dérivé de l'étain jusqu'à obtention du dihydro-2,3 diméthyl-2,2 hydroxy-7 benzofuranne.

6. Procédé selon l'une des revendications 1 à 5, caractérisé en ce que le dérivé de l'étain est un composé de formule

$$Sn\ (R)_m$$

dans laquelle R représente un atome, ou un radical minéral ou organique, et m est égal à 2 ou à 4 étant entendu que les substituants R peuvent être soit identiques soit différents, ou un réactif à base d'étain susceptible de former in situ ce composé dans les conditions de la réaction

7. Procédé selon la revendication 6, caractérisé en ce que R représente un atome d'halogène ou un radical alkyle éventuellement substitué, aryle éventuellement substitué, alkaryle éventuellement substitué ou le radical $-OR_1$ dans lequel $R_1$ représente un reste organique.

8. Procédé selon l'une des revendications 6 à 7, caractérisé en ce que m est égal à 2.

9. Procédé selon l'une des revendications 1 à 8, caractérisé en ce que le dérivé de l'étain utilisé comme catalyseur est le chlorure stanneux.

10. Procédé selon l'une des revendications 1 à 9, caractérisé en ce qu'on utilise au moins 0,00005 mole de dérivé de l'étain par mole de composé à transformer.

11. Procédé selon l'une des revendications 1 à 9, caractérisé en ce qu'on utilise de 0,0001 à 0,3 mole de dérivé de l'étain par mole de composé à transformer.

12. Procédé selon l'une des revendications 1 à 9, caractérisé en ce qu'on utilise de 0,005 à 0,1 mole de dérivé de l'étain par mole de composé à transformer.

13. Procédé selon l'une des revendications 1 à 12, caractérisé en ce que le chauffage en présence du dérivé de l'étain est effectué à une température comprise entre 60 °C et 200 °C.

14. Procédé selon l'une des revendications 1 à 11, caractérisé en ce que la transformation selon l'invention est effectuée dans un solvant organique.

**Description pour les états contractants: DE, FR, IT**

L'invention concerne un procédé de préparation du dihydro-2,3 diméthyl-2,2 hydroxy-7 benzofuranne à partir de l'orthométhallyloxyphénol.

Le dihydro-2,3 diméthyl-2,2 hydroxy-7 benzofuranne, désigné dans ce qui suit par DDHB, a pour formule:

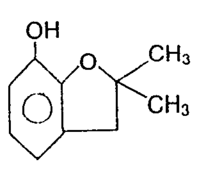

DDHB

Ce composé, en soi connu, est utilisable pour la préparation du méthylcarbamate de dihydro-2,3 diméthyl-2,2 benzofurannyl-7, insecticide polyvalent connu sous le nom de carbofuran.

L'orthométhallyloxyphénol, ou méthallyloxy-2 phénol, est le composé de formule:

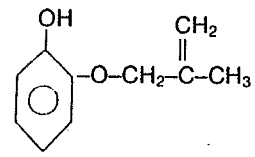

ORTHOMETHALLYLOXYPHENOL

Il est connu de préparer le DDHB à partir de l'orthométhallyloxyphénol par réarrangement de ce composé (transposition en ortho du radical méthallyle), formant intermédiairement un composé réarrangé (notamment orthométhallylpyrocatéchine) puis cyclisation de ce composé réarrangé, pour donner le DDHB.

L'orthométhallylpyrocatéchine, ou dihydroxy-1,2 méthallyl-3 benzène, est le composé de formule:

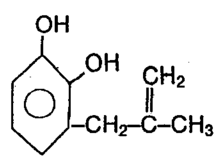

La mise en œuvre de cette transformation de l'orthométhallyloxyphénol en DDHB, par réarrangement/cyclisation a fait l'objet de plusieurs dépôts de brevets, conduisant à des résultats sensiblement différents selon les conditions utilisées:

– FR-A-1 403 952 décrit cette transformation effectuée par chauffage de l'orthométhallyloxyphénol, dans la masse, à 190 °C, la température atteignant 270 °C pendant la transformation. D'après l'essai décrit dans ce brevet le rendement n'est que de 48,2%;

– la demande de brevet européen no 79 420 062.6 (Numéro de publication: 0 012 096) décrit cette transformation effectuée par chauffage de l'orthométhallyloxyphénol à une température de 200 °C environ, en présence d'eau et éventuellement d'un solvant organique inerte. Les rendements obtenus sont de l'ordre de 69 à 72%;

– la demande de brevet européen no 80 420 130.9 (Numéro de publication 0 030 511) décrit cette transformation effectuée par chauffage de l'orthométhallyloxyphénol, à des températures en général de l'ordre de 120 à 150 °C, en présence, comme catalyseur, d'un dérivé de l'aluminium, de préférence l'isopropylate d'aluminium et éventuellement en présence d'un solvant organique. Les rendements obtenus varient de 60 à 77% environ. L'exemple 9 de cette demande de brevet européen montre que lorsqu'on opère à température plus basse (100 °C), on obtient un rendement en DDHB sensiblement plus faible (60,7%), malgré l'utilisation de quantités plus importantes de catalyseur (0,3 mole de catalyseur par mole d'orthométhallyloxyphénol);

– la demande de brevet européen no 80 104 521.2 (Numéro de publication 0 025 843)

décrit cette transformation effectuée par chauffage de l'orthométhallyloxyphénol, à des températures de 150 à 190 °C environ, dans un solvant constitué par un polyhydroxyalkyléther contenant au moins un groupe OH, en présence, comme catalyseur, d'acide p-toluène-sulfonique ou de chlorure ferrique. Selon les exemples décrits, les rendements varient de 65 et 70%.

On a par ailleurs aussi décrit (EP-A-90 679 publiée le 5 octobre 1983) la préparation de DDHB à partir d'orthométhallyloxyphénol en présence de $SnCl_4$. Dans ce document les états contractants désignés sont DE, FR et IT. En ce qui concerne ces trois états contractants, l'utilisation de $SnCl_4$ est exclue de la présente demande.

Aucun des procédés décrits dans ces références n'a toutefois donné pleinement satisfaction.

Un but de l'invention est de fournir un procédé amélioré de préparation du DDHB grâce à l'utilisation de catalyseurs différents de ceux décrits ou suggérés dans les brevets ou demandes de brevets cités plus haut.

Plus précisément, un but de la présente invention est de fournir un procédé de préparation du DDHB à partir de l'orthométhallyloxyphénol permettant d'obtenir de bons rendements en DDHB tout en opérant à une température sensiblement moins élevée que les procédés décrits dans les brevets ou demandes de brevets cités plus haut.

Il a maintenant été trouvé que ce but pouvait être atteint grâce à un nouveau procédé faisant l'objet de la présente invention.

Ce procédé concerne la préparation du DDHB à partir de l'orthométhallyloxyphénol, par chauffage, comportant un réarrangement de l'orthométhallyloxyphénol puis une cyclisation du composé réarrangé. Il est caractérisé en ce que au moins l'étape de cyclisation est effectuée par chauffage en présence d'une quantité efficace d'un catalyseur constitué par un dérivé de l'étain, qui n'est pas $SnCl_4$.

Selon un premier mode de réalisation de l'invention, l'orthométhallyloxyphénol est d'abord chauffé à une température élevée en l'absence de dérivé de l'étain et il se produit alors, selon les conditions utilisées, une conversion, partielle ou totale, du composé de départ en composé réarrangé (orthométhallylpyrocatéchine notamment). Le mélange réactionnel ainsi obtenu est ensuite chauffé en présence du dérivé de l'étain de manière à effectuer la cyclisation du dérivé réarrangé.

Selon ce premier mode de réalisation de l'invention, l'étape de réarrangement et l'étape de cyclisation peuvent être effectuées en deux opérations tout à fait distinctes: on peut ainsi chauffer l'orthométhallyloxyphénol de façon à provoquer son réarrangement, par exemple en opérant selon les conditions décrites dans la demande de brevet européen no 80 420 130.9 (page 9 essai comparatif dans EP-A-30 511) et obtenir ainsi un mélange comprenant principalement de l'orthométhallylpyrocatéchine, puis dans une deuxième opération distincte de la précédente, chauffer ce mélange réactionnel en présence du dérivé de l'étain comme défini ci-dessus. Selon une variante de ce premier mode de réalisation de l'invention, on peut également effectuer successivement dans un même dispositif le réarrangement et la cyclisation en effectuant d'abord le chauffage en l'absence du dérivé de l'étain, puis en le poursuivant en présence du dérivé de l'étain que l'on ajoute alors au mélange réactionnel.

Selon un mode de réalisation préférentiel de l'invention, le réarrangement de l'orthométhallyloxyphénol s'effectue en chauffant ce composé en présence du dérivé de l'étain et la cyclisation du composé réarrangé est également effectuée par chauffage en présence du dérivé de l'étain. Selon ce mode de réalisation préférentiel de l'invention, il est particulièrement avantageux de chauffer l'orthométhallyloxyphénol en présence du dérivé de l'étain et de poursuivre ce chauffage en présence du même dérivé de l'étain jusqu'à ce qu'il y ait formation du DDHB. Dans ces conditions, on observe que les deux transformations successives s'enchaînent immédiatement. On obtient ainsi un procédé permettant de produire le DDHB, en une seule étape, à partir de l'orthométhallyloxyphénol. Ce procédé est caractérisé en ce que l'orthométhallyloxyphénol est chauffé en présence d'un dérivé de l'étain. Il constitue le mode de réalisation préféré de l'invention.

Par dérivé de l'étain, au sens du présent texte, on entend les dérivés organiques ou minéraux de l'étain divalent et de l'étain tétravalent ainsi que tout réactif à base d'étain susceptible de former in situ, dans les conditions de la réaction un dérivé de l'étain divalent ou de l'étain tétravalent, qui n'est pas $SnCl_4$.

Ces dérivés de l'étain divalent et de l'étain tétravalent répondent à la formule générale:

$$Sn\ (R)_m$$

dans laquelle R représente un atome par exemple d'halogène, d'oxygène ou de soufre, ou un radical minéral ou organique, et m un nombre entier égal à 2 ou à 4 étant entendu que les substituants R peuvent être soit identiques soit différents à l'exception de $SnCl_4$.

Avantageusement R est choisi parmi les substituants mentionnés ci-après:

- les atomes d'halogène (de préférence de chlore si m ne représente pas un nombre égal à 4),
- les radicaux alkyle (par exemple éthyle, méthyle, butyle) ou cycloalkyle éventuellement substitués (par exemple par un ou plusieurs halogènes),
- les radicaux aryle ou alkaryle (par exemple le radical phényle) éventuellement substitués,
- les radicaux $-OR_1$ dans lesquels $R_1$ représente un reste organique tel que par exemple un radical alkyle ou aryle ou acyle, ou le radical benzofuryle, ces radicaux pouvant être éventuellement substitués (par exemple le radical dihydro-2,3 diméthyl-2,2 benzofuryle-7 ou le radical orthométhallyloxyphényle, ou les radicaux hydroxy(méthallyl)phényle, ces divers radicaux pouvant éventuellement être formés in situ).

Ainsi lorsque R représente le radical $-OR_1$, ce substituant R peut, entre autres, signifier le radical phénoxy ou le radical acétyloxy ($CH_3COO$).

Parmi les composés répondant à la formule ci-dessus, on peut utiliser selon l'objet de l'invention avantageusement des composés tels que $SnCl_2$, le méthylate d'étain, le diacétate d'étain, le diacétate de dibutylétain, le dichlorodibutylétain, cette liste n'étant pas limitative.

De préférence, on utilise comme dérivé de l'étain les dérivés de l'étain divalent, c'est-à-dire les composés répondant à la formule générale indiquée plus haut, dans laquelle m est égal à 2, R ayant la même signification que précédemment.

Parmi ces composés préférés, le chlorure stanneux $SnCl_2$ est tout particulièrement préféré, car il permet d'obtenir des rendements élevés dès la température de 100 °C, comme le montrent les exemples décrits plus bas.

La quantité de dérivé de l'étain à utiliser doit être suffisante pour permettre à la transformation de s'effectuer de façon satisfaisante dans les conditions utilisées. Pour cela on utilise généralement au moins 0,00005 mole de dérivé de l'étain par mole de composé à transformer (orthométhallyloxyphénol ou dérivé de réarrangement de ce composé). La limite supérieure de la quantité de catalyseur n'est pas critique. En pratique, on utilise avantageusement de 0,0001 à 0,3 mole de dérivé de l'étain par mole de composé à transformer et de préférence de 0,005 à 0,1 mole de dérivé de l'étain par mole de composé à transformer.

La température à laquelle est effectué le chauffage en présence du dérivé de l'étain peut être comprise entre 60 °C et 200 °C, elle est de préférence comprise entre 80 °C et 130 °C.

Lorsque le dérivé de l'étain utilisé comme catalyseur est le chlorure stanneux, des résultats tout à fait avantageux sont obtenus en opérant dès la température de 100 °C.

La transformation selon l'invention peut être effectuée par chauffage de l'orthométhallyloxyphénol et/ou de son produit de réarrangement dans la masse en l'absence de solvant.

De préférence toutefois, cette transformation est effectuée dans un solvant organique avantageusement chosi parmi les hydrocarbures aromatiques tels que le toluène, les o-, m- et p-ylènes; les hydrocarbures aliphatiques tels que l'octane ou le décane; les hydrocarbures aromatiques chlorés; les hydrocarbures cycloaliphatiques tels que le cyclohéxane; les éthers aliphatiques ou aromatiques tels que l'anisole; les cétones telles que la méthylisobutylcétone; les alcanols tels que les alcools inférieurs comme par exemple l'isopropanol, les phénols et plus particulièrement le phénol proprement dit, les polyhydroxyalkyléthers (tels que mentionnés dans la demande de brevet européen no 80 104 521.2 = EP-A-25 843) cette liste n'étant pas limitative.

La transformation selon l'invention s'effectue avantageusement sous pression atmosphérique ordinaire. Elle peut toutefois être effectuée sous une pression différente de la pression atmosphérique ordinaire. Ainsi, lorsqu'on désire utiliser un solvant dont la température d'ébullition, sous pression atmosphérique normale, est inférieure à la température à laquelle on désire opérer, la transformation selon l'invention peut être effectuée en opérant en autoclave, sous une pression supérieure à la pression atmosphérique.

La durée nécessaire pour effectuer la transformation selon l'invention dépend de la température utilisée, ainsi que d'autres facteurs tels que le choix du catalyseur, mais en général cette durée est d'autant plus courte que la température est plus élevée. Cette durée est généralement comprise entre 15 minutes et vingt heures.

En fin de réaction, le DDHB obtenu est séparé par tout moyen connu en soi tel que par exemple par distillation. Toutefois, pour certaines utilisations, il peut ne pas être nécessaire d'isoler le DDHB et il suffit alors de le laisser dans le milieu réactionnel qui est lui-même mis en œuvre de la manière souhaitée. Le dérivé de l'étain peut être éliminé en lavant la masse réactionnelle par une solution aqueuse d'un acide fort.

Les exemples 1–10, décrits à titre non limitatif, illustrent l'invention et montrent comment elle peut être mise en œuvre. L'exemple de référence ne fait pas partie de l'invention.

Dans ce qui suit, on désignera systématiquement par TT(ME) le taux de transformation de l'orthométhallyloxyphénol, évalué à partir de la quantité de ce composé présente dans le milieu réactionnel respectivement au début et à la fin de l'opération, et par RT(DDHB) le rendement en dihydro-2,3 diméthyl-2,2 hydroxy-7 benzofuranne (ou DDHB), calculé à partir de l'orthométhallyloxyphénol transformé.

Exemple 1

L'appareillage utilisé est constitué par un ballon de 100 ml, muni d'un réfrigérant ascendant et d'un barreau magnétique pour l'agitation.

On charge successivement dans ce ballon:

- 1,0294 g d'orthométhallyloxyphénol ($6{,}28.10^{-3}$ mole)
- 0,052 g de chlorure stanneux ($2{,}74.10^{-4}$ mole)
- 50 ml de méthylisobutylcétone.

Ce mélange est chauffé pendant 16 heures 30 à 100 °C, sous agitation, puis refroidi à température ambiante (25 °C). On ajoute alors 20 ml d'HCl 2N puis 20 ml d'acétate d'éthyle, et on agite l'ensemble dans une ampoule à décanter.

Après séparation de la phase organique et de la phase aqueuse, on lave la phase organique par 30 ml d'eau distillée que l'on réunit ensuite à la phase aqueuse. Cette phase aqueuse est à son tour lavée par 20 ml d'acétate d'éthyle qu'on joint à la première phase organique. On ajuste alors le volume de la phase organique à 100 ml et la solution obtenue est analysée par chromatographie en phase liquide.

On y dose:

- 0,0224 g d'orthométhallyloxyphénol non transformé ($1{,}36.10^{-4}$ mole)
- 0,793 g de DDHB ($4{,}83.10^{-3}$ mole)
- 0,0017 g d'orthométhallylpyrocatéchine ($1.10^{-5}$ mole).

Les résultats sont les suivants:
- TT(ME) = 98%
- RT(DDHB) = 78,7%.

Exemple 2

On opère selon la même méthode qu'à l'exemple 1 en utilisant le même dérivé d'étain ($SnCl_2$) et le même solvant que dans cet exemple 1, à partir de:
- 1,9421 g d'orthométhallyloxyphénol ($1,184.10^{-2}$ mole)
- 0,1121 g de chlorure stanneux ($5,91.10^{-4}$ mole)
- 35 ml de méthylisobutylcétone

et en effectuant le chauffage à 105 °C, comme à l'exemple no 1, mais pendant une durée de 9 heures 30.

Les résultats sont les suivants:
- TT(ME) = 100%
- RT(DDHB) = 79%.

Exemple 3

On opère selon la même méthode qu'à l'exemple 1, en utilisant le même dérivé d'étain et le même solvant que dans cet exemple 1, à partir de:
- 2,040 g d'orthométhallyloxyphénol ($12,44.10^{-3}$ mole)
- 0,101 g de chlorure stanneux ($5,3.10^{-4}$ mole)
- 18 ml de méthylisobutylcétone.

Le chauffage est effectué à 105 °C, pendant 6 heures, sous agitation, en atmosphère inerte.

Les résultats sont les suivants:
- TT(ME) = 87,7%
- RT(DDHB) = 79,3%.

Exemple 4

On opère selon la même méthode qu'à l'exemple 1 en utilisant le même dérivé d'étain qu'à l'exemple 1 mais en remplaçant la méthylisobutylcétone par l'isopropanol et en opérant dans un autoclave, sous atmosphère inerte.

L'essai est effectué en partant de:
- 1,619 g d'orthométhallyloxyphénol ($9,87.10^{-3}$ mole)
- 0,0891 g de chlorure stanneux ($0,470.10^{-3}$ mole)
- 12 ml d'isopropanol.

Le mélange est chauffé à 100 °C pendant 16 heures.

Les résultats sont les suivants:
- TT(ME) = 54,3%
- RT(DDHB) = 79,6%.

Exemple 5

On opère selon la même méthode qu'à l'exemple 1, en utilisant le même dérivé d'étain que dans cet exemple 1, mais en chauffant en masse l'orthométhallyloxyphénol, sans utilisation de solvant.

L'essai est effectué en partant de:
- 1,4727 g d'orthométhallyloxyphénol ($8,98.10^{-3}$ mole)
- 0,0150 g de chlorure stanneux ($7,9.10^{-5}$ mole).

Le chauffage est effectué à 100 °C pendant 7 heures 30.

Les résultats sont les suivants:
- TT(ME) = 100%
- RT(DDHB) = 53%.

Exemple 6

On opère selon la même méthode qu'à l'exemple 1, mais en utilisant comme catalyseur l'acétate d'étain, de formule:

$$(CH_3COO)_2\ Sn$$

et en opérant dans le m-xylène comme solvant.

L'essai est effectué en partant de:
- 1,6456 g d'orthométhallyloxyphénol ($10,03.10^{-3}$ mole)
- 0,0487 g d'acétate d'étain ($0,206.10^{-3}$ mole)
- 11 ml de m-xylène.

Le chauffage est effectué à 120 °C, pendant 16 heures, sous atmosphère inerte.

Les résultats sont les suivants:
- TT(ME) = 86,6%
- RT(DDHB) = 67,3%

Exemple 7

On opère selon la même méthode qu'à l'exemple 1, mais en utilisant comme catalyseur le méthylate d'étain de formule:

$$(CH_3O)_2\ Sn$$

et en chauffant l'orthométhallyloxyphénol en masse, sans utilisation de solvant.

L'essai est effectué à partir de:
- 2,2740 g d'orthométhallyloxyphénol ($13,86.10^{-3}$ mole)
- 0,0273 g de méthylate d'étain ($0,15.10^{-3}$ mole).

Le chauffage est effectué à 97 °C, pendant 14 heures, sous atmosphère inerte, avec agitation.

Les résultats sont les suivants:
- TT(ME) = 63,5%
- RT(DDHB) = 61,7%.

Le méthylate d'étain a été préparé selon la méthode décrite par J.S. MORRISON et H.M. HAENDLER dans J. Inorg. Nucl. Chem. 29, 395 (1967).

Exemple 8

On opère selon la même méthode qu'à l'exemple 1, mais en utilisant comme catalyseur le diacétate de dibutylétain de formule:

$$(CH_3COO)_2\ Sn\ (C_4H_9)_2$$

et en chauffant en masse sans utilisation de solvant.

L'essai est effectué en partant de:
- 1,720 g d'orthométhallyloxyphénol ($10,49.10^{-3}$ mole)
- 0,0368 g de diacétate de dibutylétain ($0,105.10^{-3}$ mole).

Le chauffage est effectué à 95 °C pendant 15 heures 30, sous atmosphère inerte, avec agitation.

Les résultats sont les suivants:
- TT(ME): 56%
- RT(DDHB): 50%.

Exemple 9

On opère selon la même méthode qu'à l'exemple 1, mais en utilisant comme catalyseur le dichlorodibutylétain, de formule:

$$(Cl)_2\ Sn\ (C_4H_9)_2$$

et en chauffant en masse, sans utilisation de solvant.

L'essai est effectué en partant de:

- 2,020 g d'orthométhallyloxyphénol ($12,19.10^{-3}$ mole)
- 0,036 g de dichlorodibutylétain ($0,118.10^{-3}$ mole).

Le chauffage est effectué à 120 °C pendant 6 heures.

Les résultats sont les suivants:

- TT(ME): 67,5%
- RT(DDHB): 55,5%.

Exemple de référence

On opère selon la même méthode qu'à l'exemple 1, mais en utilisant comme catalyseur le chlorure stannique de formule:

$$SnCl_4$$

et en opérant comme dans l'exemple 1 dans la méthylisobutylcétone.

L'essai est effectué à partir de:

- 2,020 g d'orthométhallyloxyphénol ($12,32.10^{-3}$ mole)
- 0,147 g de chlorure stannique ($0,56.10^{-3}$ mole)
- 18 ml de méthylisobutylcétone.

Le chauffage est effectué à 105 °C pendant 6 heures sous agitation et en atmosphère inerte.

Les résultats sont les suivants:

- TT(ME): 99%
- RT(DDHB): 48%.

Exemple 10

On utilise un tube muni d'une agitation et placé sous atmosphère inerte.

Dans ce tube on place 0,1336 g d'un produit contenant 80% en poids d'orthométhallylpyrocatéchine ($0,652.10^{-3}$ mole) et 0,0043 g de diacétate de dibutylétain ($0,012.10^{-3}$ mole).

Le chauffage est effectué pendant 3 heures 30 à 130 °C.

On observe alors que la totalité de l'orthométhallylpyrocatéchine a disparu du mélange réactionnel et on obtient ainsi 0,109 g de DDHB soit un rendement en DDHB calculé par rapport à l'orthométhallylpyrocatéchine de départ égal à 100%.

L'orthométhallylpyrocatéchine a été obtenue à partir d'orthométhallyloxyphénol par chauffage à 200 °C pendant 1 heure 30, dans le cyclohexane.

Essai A

Dans le même appareillage qu'à l'exemple 1, l'orthométhallyloxyphénol a été chauffé en masse, sans utilisation de solvant et en l'absence de dérivé de l'étain, à une température de 112 °C et ce chauffage a été poursuivi à cette même température pendant 24 heures.

Les résultats sont les suivants:

- TT(ME): 43%
- RT(DDHB): 0%.

Une analyse du mélange réactionnel montre qu'il comprend principalement de l'orthométhallylpyrocatéchine (non cyclisée) et, dans une proportion moindre, de la paraméthallylpyrocatéchine (ou dihydroxy-1,2 méthallyl-4 benzène), respectivement 62,3% et 20,2% par rapport à l'orthométhallyloxyphénol transformé.

Essai B

On répète l'essai A, en chauffant en masse l'orthométhallyloxyphénol en l'absence de dérivé de l'étain mais en effectuant ce chauffage à 130 °C pendant 15 heures.

Les résultats sont les suivants:

- TT(ME): 82%
- RT(DDHB): 1,4%.

Une analyse du mélange réactionnel montre qu'il comprend principalement de l'orthométhallylpyrocatéchine (non cyclisée) et dans une proportion moindre de la paraméthallylpyrocatéchine, respectivement 61% et 22% par rapport à l'orthométhallyloxyphénol transformé.

Essai C

On répète l'essai A en chauffant l'orthométhallyloxyphénol en l'absence de dérivé de l'étain si ce n'est que l'orthométhallyloxyphénol est chauffé à 140 °C pendant 11 heures dans un solvant organique (para-xylène).

Les résultats sont les suivants:

- TT(ME): 98,2%
- RT(DDHB): 1,8%.

Une analyse du mélange réactionnel montre qu'il comprend principalement de l'orthométhallylpyrocatéchine (non cyclisée) et dans une proportion moindre de la paraméthallylpyrocatéchine.

Ces essais A, B et C montrent qu'en l'absence de dérivé de l'étain il n'y a pratiquement pas formation de DDHB pour des températures allant de 112 °C à 140 °C, puisque dans ces conditions le RT(DDHB) est compris entre 0% et 1,8%.

L'exemple C montre de plus que le réarrangement de l'orthométhallyloxyphénol peut s'effectuer de façon satisfaisante (TT-ME) = 98,2%) en l'absence de catalyseur, à condition de chauffer à une température plus élevée (140 °C).

Les essais décrits dans les exemples 1 à 10 de notre demande de brevet montrent que dans des conditions de température tout à fait similaires, et dans certains cas plus basses, la présence d'un dérivé de l'étain dans le mélange réactionnel permet à la cyclisation de s'effectuer de façon satisfaisante, puisque dans ces conditions le RT(DDHB) varie de 48% (exemple 10) à 79,6% (exemple 4).

Une comparaison des résultats obtenus avec les dérivés de l'étain divalent, décrits dans les exemples no 1 à 7 de notre demande de brevet, à ceux obtenus avec les dérivés de l'étain tétrava-lent, et décrits dans les exemples no 8 à 10 de notre demande de brevet montre que les dérivés de l'étain divalent conduisent à des RT(DDHB) en général sensiblement plus élevés que les RT(DDHB) obtenus avec les dérivés de l'étain tétravalent.

Enfin, une comparaison du résultat obtenu avec $SnCl_2$, dans l'exemple 3: RT(DDHB) = 79,3%, à celui obtenue avec $SnCl_4$ dans l'exemple de référence: RT(DDHB) = 48%, en utilisant exactement les mêmes conditions que dans l'exemple 3, met en évidence la nette supériorité de $SnCl_2$ vis-à-vis de $SnCl_4$ pour la transformation de l'orthométhallyloxyphénol en DDHB.

Par ailleurs, si l'on se réfère aux exemples no 1 et 2 de notre demande de brevet, on observera que l'utilisation de $SnCl_2$ comme catalyseur permet d'obtenir, dès la température de 100 °C, à la fois un taux de transformation de l'orthométhallyloxyphénol élevé: TT(ME) de 98% à 100% et un RT(DDHB) élevé (78,7% à 79%), c'est-à-dire des résultats sensiblement équivalents aux meilleurs résultats obtenus à des températures nettement plus élevées dans les exemples décrits dans les publications EP-A-0 012 096 et 0 030 511 citées plus haut.

**Revendications pour les états contractants: DE, FR, IT**

1. Procédé de préparation du dihydro-2,3 diméthyl-2,2 hydroxy-7 benzofuranne par chauffage, à partir de l'orthométhallyloxyphénol, par réarrangement de l'orthométhallyloxyphénol, puis cyclisation du composé, réarrangé ainsi obtenu, caractérisé en ce que au moins l'étape de cyclisation est effectuée par chauffage en présence d'une quantité efficace d'un catalyseur constitué par un dérivé de l'étain, qui n'est pas $SnCl_4$.

2. Procédé selon la revendication 1, caractérisé en ce que le réarrangement de l'orthométhallyloxyphénol est effectué par chauffage en l'absence de dérivé de l'étain puis en ce que la cyclisation du composé réarrangé est effectuée par chauffage de ce composé en présence d'un catalyseur constitué par un dérivé de l'étain.

3. Procédé selon l'une des revendications 1 et 2, caractérisé en ce que le composé réarrangé puis soumis à cyclisation est l'orthométhallylpyrocatéchine.

4. Procédé selon la revendication 1, caractérisé en ce que le réarrangement de l'orthométhallyloxyphénol est effectué par chauffage en présence d'un dérivé de l'étain, puis en ce que la cyclisation du composé réarrangé est effectuée par chauffage en présence d'un dérivé de l'étain.

5. Procédé selon la revendication 4, caractérisé en ce que l'orthométhallyloxyphénol est chauffé en présence d'un dérivé de l'étain jusqu'à obtention du dihydro-2,3 diméthyl-2,2 hydroxy-7 benzofuranne.

6. Procédé selon l'une des revendications 1 à 5, caractérisé en ce que le dérivé de l'étain est un composé de formule

$$Sn\ (R)_m$$

dans laquelle R représente un atome, ou un radical minéral ou organique, et m est égal à 2 ou à 4 étant entendu que les substituants R peuvent être soit identiques soit différents, ou un réactif à base d'étain susceptible de former in situ ce composé dans les conditions de la réaction.

7. Procédé selon la revendication 6, caractérisé en ce que R représente un atome d'halogène ou un radical alkyle éventuellement substitué, aryle éventuellement substitué, alkaryle éventuellement substitué ou le radical $-OR_1$ dans lequel $R_1$ représente un reste organique.

8. Procédé selon l'une des revendications 6 à 7, caractérisé en ce que m est égal à 2.

9. Procédé selon l'une des revendications 1 à 8, caractérisé en ce que le dérivé de l'étain utilisé comme catalyseur est le chlorure stanneux.

10. Procédé selon l'une des revendications 1 à 9, caractérisé en ce qu'on utilise au moins 0,00005 mole de dérivé de l'étain par mole de composé à transformer.

11. Procédé selon l'une des revendications 1 à 9, caractérisé en ce qu'on utilise de 0,0001 à 0,3 mole de dérivé de l'étain par mole de composé à transformer.

12. Procédé selon l'une des revendications 1 à 9, caractérisé en ce qu'on utilise de 0,005 à 0,1 mole de dérivé de l'étain par mole de composé à transformer.

13. Procédé selon l'une des revendications 1 à 12, caractérisé en ce que le chauffage en présence du dérivé de l'étain est effectué à une température comprise entre 60 °C et 200 °C.

14. Procédé selon l'une des revendications 1 à 11, caractérisé en ce que la transformation selon l'invention est effectuée dans un solvant organique.

**Claims for the Contracting States AT, BE, CH, GB, LI, LU, NL, SE**

1. A process for the preparation of 2,3-dihydro-2,2-dimethyl-7-hydroxybenzofuran from ortho-methallyloxyphenol by heating, by rearrangement of the ortho-methallyloxyphenol and then cyclisation of the rearranged compound thus obtained, wherein at least the cyclisation step is carried out by heating in the presence of an effective amount of a catalyst consisting of a tin derivative.

2. A process according to claim 1, wherein the rearrangement of the ortho-methallyloxyphenol is carried out by heating in the absence of tin derivative, and wherein the cyclisation of the rearranged compound is then carried out by heating this compound in the presence of a catalyst consisting of tin derivative.

3. A process according to one claim 1 or 2, wherein the rearranged compound which is subjected to cyclisation is ortho-methallylpyrocatechol.

4. A process according to claim 1, wherein the rearrangement of the ortho-methallyloxyphenol is carried out by heating in the presence of a tin

derivative, and wherein the cyclisation of the rearranged compound is then carried out by heating in the presence of a tin derivative.

5. A process according to claim 4, wherein the ortho-methallyloxyphenol is heated in the presence of a tin derivative until 2,3-dihydro-2,2-dimethyl-7-hydroxybenzofuran is obtained.

6. A process according to any one of claims 1 to 5, wherein the tin derivative is a compound of the formula:

$$Sn\ (R)_m$$

in which R represents a halogen atom or an inorganic or organic group and m is equal to 2 or 4, it being understood that the substituents R can be either identical or different, or a tin-based reagent capable of forming this compound in situ under the reaction conditions.

7. A process according to claim 6, in which R represents a halogen atom, an optionally substituted alkyl radical, an optionally substituted aryl radical, an optionally substituted alkaryl radical or the radical $-OR_1$ wherein $R_1$ represents an organic radical.

8. A process according to claims 6 or 7, wherein m is equal to 2.

9. A process according to any one of claims 1 to 8, wherein the tin derivative used as the catalyst is stannous chloride.

10. A process according to any one of claims 1 to 9, wherein at least 0.00005 mol of tin derivative is used per mol of compound to be converted.

11. A process according to any one of claims 1 to 9, wherein from 0.0001 to 0.3 mol of tin derivative is used per mol of compound to be converted.

12. A process according to any one of claims 1 to 9, wherein from 0.005 to 0.1 mol of tin derivatives is used per mol of compound to be converted.

13. A process according to any one of claims 1 to 12, wherein heating in the presence of the tin derivative is carried out at a temperature of between 60 °C and 200 °C.

14. A process according to any one of claims 1 to 13, wherein the conversion according to the invention is carried out in an organic solvent.

**Claims for the Contracting States DE, FR, IT**

1. A process for the preparation of 2,3-dihydro-2,2-dimethyl-7-hydroxybenzofuran from ortho-methallyloxyphenol by heating, by rearrangement of the ortho-methallyloxyphenol and then cyclisation of the rearranged compound thus obtained, wherein at least the cyclisation step is carried out by heating in the presence of an effective amount of a catalyst consisting of a tin derivative which is not $SnCl_4$.

2. A process according to claim 1, wherein the rearrangement of the ortho-methallyloxyphenol is carried out by heating in the absence of tin derivative, and wherein the cyclisation of the rearranged compound is then carried out by heating this compound in the presence of a catalyst consisting of tin derivative.

3. A process according to one claim 1 or 2, wherein the rearranged compound which is subjected to cyclisation is ortho-methallylpyrocatechol.

4. A process according to claim 1, wherein the rearrangement of the ortho-methallyloxyphenol is carried out by heating in the presence of a tin derivative, and wherein the cyclisation of the rearranged compound is then carried out by heating in the presence of a tin derivative.

5. A process according to claim 4, wherein the ortho-methallyloxyphenol is heated in the presence of a tin derivative until 2,3-dihydro-2,2-dimethyl-7-hydroxybenzofuran is obtained.

6. A process according to any one of claims 1 to 5, wherein the tin derivative is a compound of the formula:

$$Sn\ (R)_m$$

in which R represents a halogen atom or an inorganic or organic group and m is equal to 2 or 4, it being understood that the substituents R can be either identical or different, or a tin-based reagent capable of forming this compound in situ under the reaction conditions.

7. A process according to claim 6, in which R represents a halogen atom, an optionally substituted alkyl radical, an optionally substituted aryl radical, and optionally substituted alkaryl radical or the radical $-OR_1$ wherein $R_1$ represents an organic radical.

8. A process according to claims 6 or 7, wherein m is equal to 2.

9. A process according to any one of claims 1 to 8, wherein the tin derivative used as the catalyst is stannous chloride.

10. A process according to any one of claims 1 to 9, wherein at least 0.00005 mol of tin derivative is used per mol of compound to be converted.

11. A process according to any one of claims 1 to 9, wherein from 0.0001 to 0.3 mol of tin derivative is used per mol of compound to be converted.

12. A process according to any one of claims 1 to 9, wherein from 0.005 to 0.1 mol of tin derivative is used per mol of compound to be converted.

13. A process according to any one of claims 1 to 12, wherein heating in the presence of the tin derivative is carried out at a temperature of between 60 °C and 200 °C.

14. A process according to any one of claims 1 to 13, wherein the conversion according to the invention is carried out in an organic solvent.

**Patentansprüche für die Vertragsstaaten AT, BE, CH, GB, LI, LU, NL, SE**

1. Verfahren zur Herstellung von 2,3-Dihydro-2,2-dimethyl-7-hydroxybenzofuran durch Erhitzen von o-Methallyloxyphenol unter Umlagerung und anschliessender Cyclisierung der auf diese Weise erhaltenen umgelagerten Verbindung, gekennzeichnet dadurch, dass mindestens die Cyclisierung in Anwesenheit einer wirksamen Menge einer Zinnverbindung als Katalysator durchgeführt wird.

2. Verfahren nach Punkt 1, gekennzeichnet dadurch, dass die Umlagerung des o-Methallyloxyphenols in Abwesenheit der Zinnverbindung durchgeführt wird und anschliessend die Cyclisierung der umgelagerten Verbindung in Anwesenheit einer Zinnverbindung als Katalysator erfolgt.

3. Verfahren nach einem der Punkte 1 und 2, gekennzeichnet dadurch, dass die umgelagerte Verbindung, die anschliessend der Cyclisierung unterworfen wird, o-Methallylbrenzcatechin ist.

4. Verfahren nach Punkt 1, gekennzeichnet dadurch, dass auch die Umlagerung des o-Methallyloxyphenols in Anwesenheit einer Zinnverbindung durchgeführt wird.

5. Verfahren nach Punkt 4, gekennzeichnet dadurch, dass o-Methallyloxyphenol in Anwesenheit einer Zinnverbindung erhitzt wird, bis man 2,3-Dihydro-2,2-dimethyl-7-hydroxybenzofuran erhält.

6. Verfahren nach einem der Punkte 1 bis 5, gekennzeichnet dadurch, dass die Zinnverbindung eine Verbindung der Formel

$$Sn(R)_m$$

ist, in der R ein Atom oder eine anorganische oder organische Gruppe darstellt, m gleich 2 oder 4 ist und die Substituenten R gleich oder verschieden sein können, oder ein Reaktionspartner auf der Basis von Zinn, der unter den Reaktionsbedingungen in situ diese Verbindung bilden kann.

7. Verfahren nach Punkt 6, gekennzeichnet dadurch, dass R ein Halogenatom oder eine gegebenenfalls substituierte Alkyl-, Aryl- oder Alkarylgruppe oder die Gruppe $-OR_1$ darstellt, in der $R_1$ einen organischen Rest bedeutet.

8. Verfahren nach einem der Punkte 6 und 7, gekennzeichnet dadurch, dass m gleich 2 ist.

9. Verfahren nach einem der Punkte 1 bis 8, gekennzeichnet dadurch, dass die als Katalysator verwendete Zinnverbindung Zinn(II)-chlorid ist.

10. Verfahren nach einem der Punkte 1 bis 9, gekennzeichnet dadurch, dass man mindestens 0,00005 mol Zinnverbindung pro mol umzuwandelnde Verbindung verwendet.

11. Verfahren nach einem der Punkte 1 bis 9, gekennzeichnet dadurch, dass man 0,0001 bis 0,3 mol Zinnverbindung pro mol umzuwandelnde Verbindung verwendet.

12. Verfahren nach einem der Punkte 1 bis 9, gekennzeichnet dadurch, dass man 0,005 bis 0,1 mol Zinnverbindung pro mol umzuwandelnde Verbindung verwendet.

13. Verfahren nach einem der Punkte 1 bis 12, gekennzeichnet dadurch, dass man in Anwesenheit der Zinnverbindung auf eine Temperatur zwischen 60 und 200 °C erhitzt.

14. Verfahren nach einem der Punkte 1 bis 11, gekennzeichnet dadurch, dass die Umwandlung in einem organischen Lösungsmittel durchgeführt wird.

**Patentansprüche für die Vertragsstaaten DE, FR, IT**

1. Verfahren zur Herstellung von 2,3-Dihydro-2,2-dimethyl-7-hydroxybenzofuran durch Erhitzen von o-Methallyloxyphenol unter Umlagerung und anschliessender Cyclisierung der auf diese Weise erhaltenen umgelagerten Verbindung, gekennzeichnet dadurch, dass mindestens die Cyclisierung in Anwesenheit einer wirksamen Menge einer Zinnverbindung als Katalysator ausser $SnCl_4$ durchgeführt wird.

2. Verfahren nach Punkt 1, gekennzeichnet dadurch, dass die Umlagerung des o-Methallyloxyphenols in Abwesenheit der Zinnverbindung durchgeführt wird und anschliessend die Cyclisierung der umgelagerten Verbindung in Anwesenheit einer Zinnverbindung als Katalysator erfolgt.

3. Verfahren nach einem der Punkte 1 und 2, gekennzeichnet dadurch, dass die umgelagerte Verbindung, die anschliessend der Cyclisierung unterworfen wird, o-Methallylbrenzcatechin ist.

4. Verfahren nach Punkt 1, gekennzeichnet dadurch, dass auch die Umlagerung des o-Methallyloxyphenols in Anwesenheit einer Zinnverbindung durchgeführt wird.

5. Verfahren nach Punkt 4, gekennzeichnet dadurch, dass o-Methallyloxyphenol in Anwesenheit einer Zinnverbindung erhitzt wird, bis man 2,3-Dihydro-2,2-dimethyl-7-hydroxybenzofuran erhält.

6. Verfahren nach einem der Punkte 1 bis 5, gekennzeichnet dadurch, dass die Zinnverbindung eine Verbindung der Formel

$$Sn(R)_m$$

ist, in der R ein Atom oder eine anorganische oder organische Gruppe darstellt, m gleich 2 oder 4 ist und die Substituenten R gleich oder verschieden sein können, oder ein Reaktionspartner auf der Basis von Zinn, der unter den Reaktionsbedingungen in situ diese Verbindung bilden kann.

7. Verfahren nach Punkt 6, gekennzeichnet dadurch, dass R ein Halogenatom oder eine gegebenenfalls substituierte Alkyl-, Aryl- oder Alkarylgruppe oder die Gruppe $-OR_1$ darstellt, in der $R_1$ einen organischen Rest bedeutet.

8. Verfahren nach einem der Punkte 6 und 7, gekennzeichnet dadurch, dass m gleich 2 ist.

9. Verfahren nach einem der Punkte 1 bis 8, gekennzeichnet dadurch, dass die als Katalysator verwendete Zinnverbindung Zinn(II)-chlorid ist.

10. Verfahren nach einem der Punkte 1 bis 9, gekennzeichnet dadurch, dass man mindestens 0,00005 mol Zinnverbindung pro mol umzuwandelnde Verbindung verwendet.

11. Verfahren nach einem der Punkte 1 bis 9, gekennzeichnet dadurch, dass man 0,0001 bis 0,3 mol Zinnverbindung pro mol umzuwandelnde Verbindung verwendet.

12. Verfahren nach einem der Punkte 1 bis 9, gekennzeichnet dadurch, dass man 0,005 bis 0,1 mol Zinnverbindung pro mol umzuwandelnde Verbindung verwendet.

13. Verfahren nach einem der Punkte 1 bis 12, gekennzeichnet dadurch, dass man in Anwesenheit der Zinnverbindung auf eine Temperatur zwischen 60 und 200 °C erhitzt.

14. Verfahren nach einem der Punkte 1 bis 11, gekennzeichnet dadurch, dass die Umwandlung in einem organischen Lösungsmittel durchgeführt wird.